# EUROPEAN PATENT APPLICATION

(11) **EP 4 012 017 A1**
(43) Date of publication of application: **15.06.2022**
(21) Application number: 20850981.0
(22) Date of filing: 03.08.2020
(51) Int. Cl.: C12N 1/04, C12N 1/20, A23K 10/16, A23L 33/135

(54) **METHOD FOR PREPARING PURE PLANT-BASED MICROBIAL CULTURE**

(30) Priority: 06.08.2019 KR 20190095469
(71) Applicant: Gon Co., Ltd, Seoul 06193 (KR)
(72) Inventor: SUN, Dae-san, Daejeon 34895 (KR)
(74) Representative: Jacobi, Markus Alexander
(86) International application number: PCT/KR2020/010197
(87) International publication number: WO 2021/025405

(57) **Abstract**

The present invention relates to a method for preparing a pure plant-based microbial culture, and provides: an application of a culture technology using both plant-derived juice and dietary fiber materials; and a culture and processing process for processing plant-derived vitamins and functional polysaccharides in a usable form, and adsorbs a microbial culture on a pure plant-based dietary fiber so as to maximally inhibit use in the oral cavity and digestive organs by harmful bacteria and selectively utilize microbial culture for the growth of beneficial bacteria, and thus, the present invention forms beneficial bacteria-oriented microcosms by strongly inducing the selective growth of beneficial bacteria preferentially in microcosms in the oral cavity and digestive organs.

## Description

### [Technical Field]

The present invention relates to a method for production of a culture of pure plant microorganisms, and more particularly, which includes: forming a culture of pure plant-based microbes using dietary fibers and polysaccharide materials such as fruits and vegetables as a carrier serving as a preservative and coating component for microbes, whereby the culture can be utilized as a raw material that serves as a medium to increase the preservation of microbes, and intestinal expandability and performance for settlement of microbes in the animal and/or human body, and the culture may influence on growth improvement, preservation improvement, and inclusion and even preservation of substances such as vitamins by fixing the culture between textures of the dietary fibers from the time of microbial cultivation, thereby producing a new high-quality microbial culture.

### [Background Art]

Existing microbial cultures such as lactic acid bacteria for food, cosmetics and feed are mostly used in products by incubating the bacteria using lactose and milk protein, and then converting the culture product into a form capable of long-term storage and inoculation through a freeze-drying method (or lyophilization).

Whole milk powder, milk casein, etc., which are mainly used in the lyophilization of existing lactic acid bacteria, are animal raw materials, while vegetable lactic acid bacteria can be used during seed cultivation. However, strictly mentioned, the above culture is not considered as a pure plant culture since this is not a product obtained by inoculating the seed on a plant medium and fermenting the same.

Further, there are some cases in which, after culturing using soy protein, the product is further prepared in the form of a liquid lactobacillus beverage. However, this is not an example utilizing lyophilization and, if lyophilization is to be performed, a binder or protein as a material to prevent destruction of live bacteria may be used, therefore, it is also difficult that the resulting product refers to a pure plant culture.

Meanwhile, a number of studies have demonstrated that intestinal bacteria or microbial groups settled in the body are more easily survived by dietary fibers or vegetable polysaccharides, and the dietary fibers or vegetable polysaccharides are more effective for construction of environments for settlement, and therefore, research and development related to the culture and production described above are still required.

### [Disclosure]

### [Technical Problem]

In order to solve the above problems, an object of the present invention is to provide a new type of microbial culture, which is obtained using vegetable raw materials only, according to an optimum incubation and processing method such that generation of bio-colonies is induced by directly adsorbing and growing microbes on dietary fibers or dietary fiber-derived polysaccharides, and the bio-colonies are used in a liquid state as it is or through freeze-drying so as to greatly improve a survival rate in the stomach and intestines while maximizing the growth and settlement thereof in the intestines.

### [Technical Solution]

The method for production of a pure plant-based microbial culture according to the present invention may include: a crushing process of fruits and vegetables of a plant to a size of about 0.01 to 10 mm;
an extraction and separation process to extract fruit juice from the crushed fruits or vegetables in a pressurized or reduced pressure extraction manner, while filtering and separating fibrous components;
a sterilization process to disinfect microbes from the juice extracted and separated in the extraction separation process through sterilization;
a culturing process that includes: adding moisture to the sterilized fruit juice to adjust a sugar content to a range of brix 1 to 25; inoculating the same with lactic acid bacteria, bacillus and yeast; and adding 0.01 to 100 g of yeast extract, 0.001 to 10 g of vitamin complex, 0.01 to 100 g of calcium carbonate, 0.01 to 100 g of sodium hydrogen carbonate, 0.01 to 100 g of magnesium chloride and 0.01 to 100 g of zinc gluconate to 1 liter of culture broth, followed by incubating the mixture for 12 to 120 hours;
a mixing and culturing process that includes preparing the culture broth cultured in the above culturing process and finely ground ("pulverized") dry fiber powder and then admixing the same in a relative ratio of 1:1 to 1:1.5, followed by incubation for 1 to 48 hours in order to induce formation of bio-colonies in the dietary fiber and polysaccharide binder undergoing re-cultivation; and
a freeze-drying process of the culture product blended and re-cultured in the above mixing and culturing process S5.

### [Advantageous effects]

As described above, the present invention may provide a new processing method of syn-biotics that have all features of microbial probiotics as well as prebiotics of dietary fiber and plant-derived components, the method including: firstly using both of liquid and dietary fiber components extracted from plants as a raw material for culturing live bacteria in a processing stage; culturing all raw materials except for other minerals and vitamin complex components with using the plant-derived components; using the dietary fiber as a binder and adsorbing microbes thereto, followed by incubation thereof; and utilizing the culture product as a binder or a fixing material in freeze-dried state to thus produce a complete pure plant-based culture. Since the produced syn-biotics have properties capable of easily dissolved in water, the present invention is beneficial with an advantage that it can be utilized in other processing methods using the above syn-biotics.

Further, the present invention may use dietary fibers and polysaccharide materials such as fruits and vegetables as a carrier that serves as a preservative and coating component, thereby producing a pure plant-based microbial culture. The produced culture may be utilized as a raw material serving as a medium to improve preservation, intestinal expandability and a performance for settlement of microbes in the animal and/or human body, and the culture may influence on growth improvement, preservation improvement, and inclusion and even preservation of substances such as vitamins by fixing the culture between textures of the dietary fibers from the time of microbial cultivation, therefore, the present invention is beneficial with an advantage of producing a new high-quality microbial culture.

### [Description of Drawings]

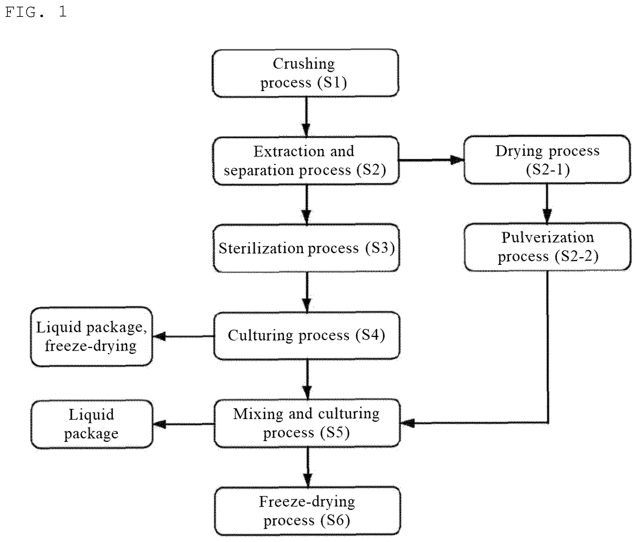

FIG. 1 is a block diagram showing an embodiment of the present invention.
FIG. 2(a) is the first page of the test report illustrating the result of test for antagonism of syn-biotics according to the present invention.
FIG. 2(b) is the second page of the test report illustrating the result of test for antagonism of syn-biotics according to the present invention.
FIG. 3 illustrates the result of test for application of syn-biotics according to the present invention in the oral cavity.

### [Detailed Description of Preferred Embodiments of Invention]

First of all, when adding reference numerals to elements in the drawings, it should be noted that the same elements have the same numerals as possible even if they are indicated on different drawings.

Further, in the description of the present invention, if it is considered that a detailed description of related known configuration or function may obscure the subject matter of the present invention, a detailed description thereof will be omitted.

Hereinafter, preferred embodiments of the present invention will be described in detail with reference to the accompanying drawings.

### Crushing process (S1)

Fruits and vegetables of a plant are crushed into a size of 0.01 to 10 mm.

### Extraction and separation process (S2)

Fruit juice is extracted from the crushed fruits or vegetables in a pressurized or reduced pressure extraction manner, and the fibrous component is filtered and separated.

### Drying process (S2-1)

Solid material of the fibrous component extracted and separated in the extraction and separation process (S2) is dried in a low temperature cold air drying manner.

At this time, in order to minimize changes in properties such as browning of the solid, the drying is conducted at 5 to 28 °C for 48 hours or more, so as to reach a moisture content of about 10 to 30%.

### Pulverization process (S2-2)

The dried solid is pulverized into fine powder

### Sterilization process (S3)

The extracted and separated fruit juice from the extraction and separation process S2 is sterilized to disinfect microbes through sterilization in pressurized and heating conditions or at low temperature under reduced pressure.

### Culturing process (S4)

Lactic acid bacteria may include one or more selected from Leuconostoc citreum, Leuconostoc lactis, Leuconostoc mesenteroides subsp. dextranicum, Leuconostoc mesenteroides subsp. mesenteroides, Leuconostoc carnosum, Leuconostoc gellidum, Leuconostoc kimchii and Leuconostoc inhae, and
one or more selected from Lactobacillus paracasei, Lactobacills casei, Lacillus acidophilus, Lactobacillus plantarum, Lactobacillus kimchii, Lactobacillus brevis, Lactobacillus delbrueckii subsp. bulgaricus, Lactobacillus delbrueckii subsp. Lactis, Lactobacillus gassari, Streptococcus salivarius, Lactobacillus reuteri, Lactobacillus ruburum, Weisella koreensis, Weisella cibaria and Weisella paramesenteroides;

Bacillus may include one or more selected from Bacillus subtillis, Bacillus subtillis natto, Bacillus amyloliquegaciens, Bacillus subtillis sakei, Bacillus polyfermenticus and Bacillus pumilus; and

Yeasts may include one or more selected from Candida mogii, Candida tropicalis, Saccharomyces cerevisiae and Saccharomyces calsbergensis,
wherein a sugar content is adjusted to a range of brix 1 to 25 by adding moisture to the sterilized fruit juice, followed by adding 0.01 to 100 g of yeast extract, 0.001 to 10 g of vitamin complex, 0.01 to 100 g of calcium carbonate, 0.01 to 100 g of sodium hydrogen carbonate, 0.01 to 100 g of magnesium chloride and 0.01 to 100 g of zinc gluconate to 1 liter of culture broth, and then incubating the mixture for 12 to 120 hours.

At this time, the incubated culture broth may be used in a liquid state or freeze-dried depending on use and finally processed forms thereof, however, is not necessarily limited thereto.

### Mixing and culturing process (S5)

The culture broth cultured in the above culturing process S4 and the pulverized dry fiber powder in the pulverization process S2 2 are admixed in a relative ratio of 1:1 to 1:1.5, followed by incubation for 1 to 48 hours in order to induce formation of biocolonies in the dietary fiber and polysaccharide binder undergoing re-cultivation.

A mixing ratio of the culture broth and the fiber powder of fruits and vegetables may vary depending upon use thereof, and the mixed culture may be used as a liquid processed product containing the dietary fiber admixed in the mixed culture.

The advantage of the above processing is that: materials such as microbes and vitamins admixed between the dietary fibers and polysaccharides may exist in the form of colloids aggregated with the mixed binder, so that a porous material may be present between the polysaccharide coatings or dietary fibers from which body fluid was drained. Therefore, the above materials may not easily become extinct by external irritating substances such as gastric juice but be smoothly supplied to the intestine, and viability is improved to thus ensure smooth intestinal supply and settlement in the intestine.

### Freeze-drying process (S6)

The mixed and re-cultivated product in the mixing and culturing process S5 is subjected to freeze-drying.

After proceeding the freeze-drying, freeze-dried solid (or lyophilisate) may be pulverized in a suitable form to be processed into powder, liquid, pill or tablet formulation useful for ingestion, thereby completing the process.

Syn-biotics which are mainly based on dietary fibers and vegetable polysaccharides may induce preferentially dominant strains that can utilize dietary fibers as a substrate among the microbes in the mouth and the digestive organ, thereby leading the growth of mainly beneficial microbes in regard to competition for food and creation of biofilms. Further, syn-biotics may serve as an assistant to make it easier for probiotic microbes used in culture to more easily come to adhesion, growth and diffusion thereof.

Further, the probiotics adsorbed to the dietary fibers through cultivation using the dietary fibers may be more easily adapted to the oral environment and digestive organs (stomach, intestine, duodenum), thereby maximizing effects of syn-biotics.

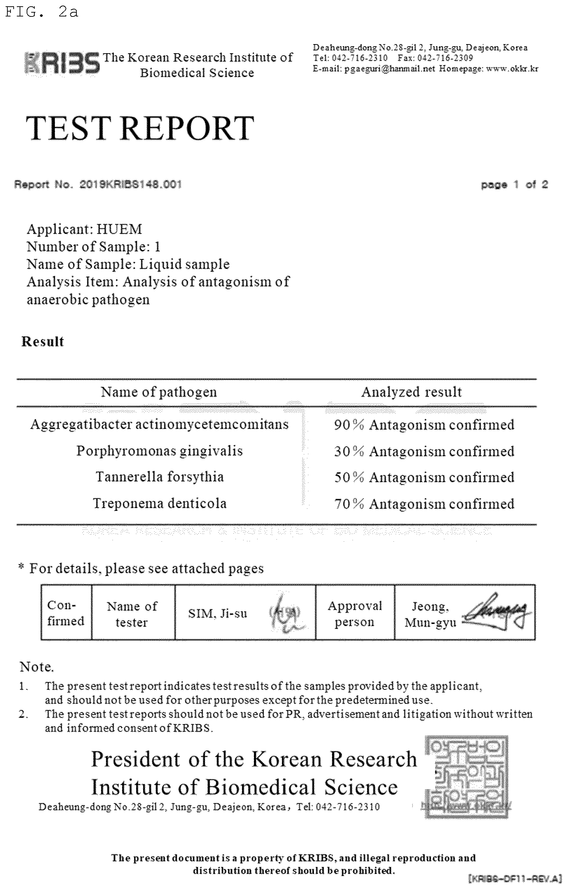

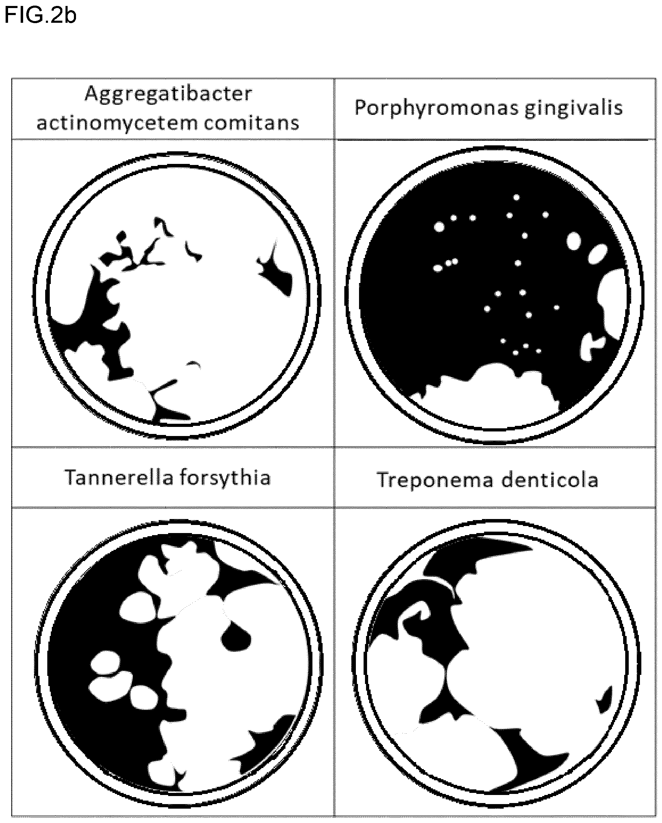

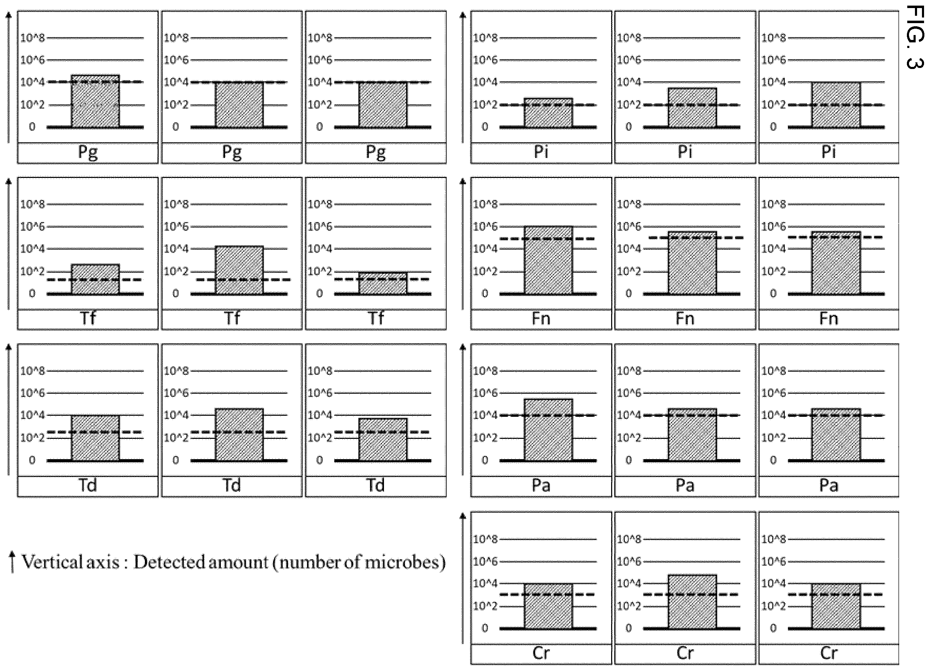

Hereinafter, a method for producing a syn-biotic culture using melon according to the present invention will be described in detail with reference to FIGS. 2 and 3.

First, among the melons cultivated in Seongju-gun, an off-grade product was washed and crushed, followed by separating fruit juice and solids using a pressurized juicer.

After adding water to the extracted juice to adjust a sugar content in the range of brix 1 to 25, yeast extract, vitamin complex, calcium carbonate, sodium hydrogen carbonate, magnesium and zinc gluconate were partially added thereto and then probiotics strain was further added, followed by incubation for 3 days to obtain a plant-based culture broth.

Even when the cultured crude broth is freeze-dried as it is, it may be processed into a product in which a large amount of dietary fibers exists. However, in order to attain maximum effects of syn-biotics, the separated solid was first sterilized and then the dried solid was sterilized under pressure and heating conditions or at a low temperature under reduced pressure. Thereafter, the sterilized product was subjected to drying while minimizing discoloration or deterioration through low temperature cold air drying, and then pulverization.

The pulverized solid may be mixed with the culture broth and transferred to a freeze-drying process as it is, otherwise, after incubation for 1 to 2 days, the freeze-drying process may be implemented.

Following this, the freeze-dried culture was crushed and transferred to a packaging process.

At this time, oligosaccharides or other sweeteners, or other dietary fiber culture prepared by the same process may be further included as additional auxiliary substances.

With regard to harmful bacteria inhibitory activity attained using the composite microbial dietary fiber syn-biotics produced above, experiments for antagonism and inhibition against *Aggretibactor actinomycetemcomitans, Porphyromonas gingivalis, Tannerella forsythia* and *Treponema denticola bacteria* were conducted.

First, each harmful strain was incubated in a stacked layer structure to create a culture layer. Then, the composite microbial dietary fiber-based syn-biotics was dissolved in purified water and cultured on the top of the stacked layer for 1 day. For the culture product, antagonism was examined for verification, and a result of the examination is shown in FIG. 2.

Accordingly, a result of examining a change after culture for 1 day is shown in FIG. 2.

Specifically, more than 90% antagonism to *Aggretibactor actinomycetemcomitans,* more than 30% antagonism to *Porphyromonas gingivalis,* more than 50% antanosim to *Tannerella forsythia,* and more than 70% direct antagonism to *Treponema denticola* were demonstrated. Further, the results of examinations that apply the culture products to the oral cavity are shown in FIG. 3.

FIG. 3 show the changes in microbes of the oral cavity after applying the syn-biotics directly to the oral cavity of a male in their late 30s three times for about 2 weeks (July 21, 2018, July 30, 2018, August 08, 2018) through PCR analysis, as shown in the table.

It could be seen in FIG. 3 that: *Aggretibactor actinomycetemcomitans* was not indicated as completely disappeared. Further, Pg (*Popyromonas gingivalis*) decreased from 10^5 to 10^4 by one tenth, and other bacteria increased with decrease of Pg, and finally, all harmful bacteria were reduced after 2 weeks.

The above experiment is an example demonstrating that the composite beneficial microbe dietary fiber culture of the present invention may suppress harmful bacteria in the oral cavity and make beneficial microbes to be preferentially dominant.

Specifically, the above results are a data proving that the present invention is very effective in creating a dominant situation of beneficial microbes in the oral cavity or intestines, in consideration of microbes in the mouth eventually associated with intestinal microbes.

As such, the above description is merely illustrative of the technical idea of the present invention, and those skilled in the art to which the present invention pertains will be able to design various modifications and variations without departing from the essential characteristics of the present invention.

Accordingly, the embodiments disclosed in the present invention are not intended to restrict the technical idea of the present invention but to explain the same, and the scope of the technical configurations of the present invention is not limited by these embodiments.

Therefore, the scope of protection of the present invention should be interpreted by the following claims, and all technical ideas within the scope equivalent thereto should be interpreted as being included in the scope of the present invention.

### Description of reference numerals

S1: Crushing process, S2: Extraction and separation process, S2-1: Drying process, S2-2: Pulverization process,
S3: Sterilization process, S4: Culturing process, S5: Mixing and culturing process, S6: Freeze-drying process

## Claims

1. A method for production of a pure plant-based microbial culture, comprising:
a crushing process of fruits and vegetables of a plant to a size of about 0.01 to 10 mm (S1);
an extraction and separation process to extract fruit juice from the crushed fruits or vegetables in a pressurized or reduced pressure extraction manner and filtering and separating fibrous components (S2);
a sterilization process to disinfect microbes from the juice extracted and separated in the extraction separation process through sterilization (S3);
a culturing process that includes: adding moisture to the sterilized fruit juice to adjust a sugar content to a range of brix 1 to 25; inoculating the same with lactic acid bacteria, bacillus and yeast; and adding 0.01 to 100 g of yeast extract, 0.001 to 10 g of vitamin complex, 0.01 to 100 g of calcium carbonate, 0.01 to 100 g of sodium hydrogen carbonate, 0.01 to 100 g of magnesium chloride and 0.01 to 100 g of zinc gluconate to 1 liter of culture broth, followed by incubating the mixture for 12 to 120 hours (S4);
a mixing and culturing process that includes preparing the culture broth obtained in the above culturing process and finely ground ("pulverized") dry fiber powder, and admixing the same in a relative ratio of 1:1 to 1:1.5, followed by incubation for 1 to 48 hours in order to induce formation of bio-colonies in the dietary fiber and polysaccharide binder undergoing re-cultivation (S5); and
a freeze-drying process of the cultured mixture blended and re-cultured in the above mixing and culturing process S5 (S6) .

2. The method according to claim 1, wherein the extraction and separation process (S2) includes: a drying process to dry solid of fibrous components extracted and separated in the extraction and separation process (S2) in a low temperature cold air drying manner (S2-1), wherein the drying is conducted at 5 to 28 °C for 48 hours or more in order to minimize a change in properties of the solid; and
a pulverization process to pulverize the dried solid into fine powder (S2-2)

3. The method according to claim 1, wherein lactic acid bacteria in the culturing process (S4) includes one or more selected from Leuconostoc citreum, Leuconostoc lactis, Leuconostoc mesenteroides subsp. dextranicum, Leuconostoc mesenteroides subsp. Mesenteroides, Leuconostoc carnosum, Leuconostoc gellidum, Leuconostoc kimchii and Leuconostoc inhae, and
one or more selected from Lactobacillus paracasei, Lactobacills casei, Lacillus acidophilus, Lactobacillus plantarum, Lactobacillus kimchii, Lactobacillus brevis, Lactobacillus delbrueckii subsp. bulgaricus, Lactobacillus delbrueckii subsp. Lactis, Lactobacillus gassari, Streptococcus salivarius, Lactobacillus reuteri, Lactobacillus ruburum, Weisella koreensis, Weisella cibaria and Weisella paramesenteroides;
Bacillus includes one or more selected from Bacillus subtillis, Bacillus subtillis natto, Bacillus amyloliquegaciens, Bacillus subtillis sakei, Bacillus polyfermenticus and Bacillus pumilus; and
Yeasts include one or more selected from Candida mogii, Candida tropicalis, Saccharomyces cerevisiae and Saccharomyces calsbergensis, and
wherein the culturing process consists of admixing the above substances and using the same for inoculation.

4. The method according to claim 1, wherein the culture broth obtained in the culturing process (S4) is used in either a liquid packaging manner or a packaging manner under freeze-dried condition depending upon use and finally processed forms of the culture broth.

5. The method according to claim 1, wherein the culture product in a colloidal form mixed and cultured in the mixing and culturing process (S5) is differently packaged depending upon use and processed forms of the product, and used as a liquid processed product.

6. The method according to claim 1, wherein the freeze-dried product of the freeze-drying process (S6) is processed into any one form of powder, liquid, pill and tablet formulations depending upon use and processed forms of the product.
